(19) Europäisches Patentamt
European Patent Office
Office européen des brevets

(11) Publication number: **0 302 147 A1**

(12) # EUROPEAN PATENT APPLICATION

(21) Application number: 87306848.0

(22) Date of filing: 03.08.87

(51) Int. Cl.[4]: A61K 31/557 , A61K 7/42 , A61K 47/00

(43) Date of publication of application: 08.02.89 Bulletin 89/06

(84) Designated Contracting States: AT BE CH DE FR GB IT LI LU NL SE

(71) Applicant: THE PROCTER & GAMBLE COMPANY
One Procter & Gamble Plaza
Cincinnati Ohio 45202(US)

(72) Inventor: Warren, Raphael
6258 Robison Road
Cincinnati Ohio 45213(US)
Inventor: Reed, Jody Claire
2905 Linwood Road
Cincinnati Ohio 45208(US)

(74) Representative: Brooks, Maxim Courtney et al
Procter & Gamble (NTC) Limited Whitley Road Longbenton
Newcastle-upon-Tyne NE12 9TS(GB)

(54) **Topical tanning compositions.**

(57) Compositions of the invention provide a safe and effective amount of an artificial tanning agent selected from prostaglandins $D_2$, $E_1$, and $E_2$ in a topical vehicle which provides enhanced penetration into and through the skin.

EP 0 302 147 A1

# TOPICAL TANNING COMPOSITIONS

## Technical Field

This invention relates to methods and compositions for tanning the skin, both with and without exposure to ultraviolet rays.

## Background of the Invention

Many persons desire to have a tanned appearance. A tanned appearance is considered to be a healthy and attractive appearance especially by younger persons.

The standard means of obtaining such an appearance is by controlled exposures to a source of ultraviolet radiation, either the sun or an artificial radiation source such as a tanning booth or bed or a combination thereof. Some persons desire a tanned appearance in the absence of such exposure. This may be for a variety of reasons: i.e., lack of time to obtain such controlled exposure, a fear of the effects of such radiation on skin or other body tissues or bodily functions, the expense of artificial radiation sources and access thereto, a desire for a tanned appearance in the winter, convenience or a combination thereof.

Artificial means for obtaining a tanned appearance may be divided into temporary and permanent means. Temporary means include primarily cosmetics such as foundations, bronzers, bases and the like and are available in the form of lotions, creams, sticks, gels, and foams. Such cosmetics suffer from several problems: expense, difficulty of application, rub-off on clothing, frequency of reapplication, lack of substantivity to water, and inconvenience.

Permanent artificial tanning means have included dyes, pigments, and pigment and/or color inducers. The early products of this type contained dihydroxyacetone, a material which combines with amino acids on the skin to form a yellow or bronze color. These products suffered from the inability of the consumer to apply the products evenly. This resulted in a streaked appearance.

Newer water-soluble FD&C dyes have been used to control streaky appearance, see, e.g. "Bronzers for the Male Market", Kalish, J. DCI, 102:38 (1968). These are generally in the form of creams or gels and employ Carbopol® resins for ease of application.

Pigment inducers such as tanning pills are also available. The pills employ antioxidants such as beta-carotene and canthaxanthin to bring out a yellow-orange "tan". These pills cause the yellow shades which plagued the early products. They may also cause color deposits in blood and body tissues. Additionally canthaxanthin may cause crystalline deposits in the eyes which affect night vision after long term use.

New products and research efforts have been directed to producing a tanned appearance by use of compounds which stimulate the skin to produce melanin (melanogenesis) in sufficient amounts to simulate the response to exposure to an ultraviolet source. Melanin is produced by cells called melanocytes which rest on the basal lamina in the dermal layer of the skin. Melanin production is a complex physical/biochemical phenomena which can be affected by many factors, internal and external. For a complete discussion of the structure and functions of the skin, see Biochemistry and Physiology of the Skin, Editor, L. Goldsmith, M.D., Oxford Press, 1983, Vols. I & II, incorporated herein by reference.

As stated above, certain compounds when introduced into skin cells will stimulate melanogenesis, the production of melanin-containing organelles called melanosomes, and dispersal thereof. Among these compounds are various peptide and protein hormones, and certain prostaglandins.

The prostaglandins have been disclosed in various published in-vitro studies, infra, all of which publications are incorporated herein by reference, to stimulate melanogenesis to some degree. J. Sauk, J.J., White, J.G., and Withop, C.V., "Influence of Prostaglandins $E_1$,$E_2$ and Arachidonate on Melansomes in Melanocytes and Keratinocytes of Anagen Hair Bulbs" Journal of Investigative Dermatology, Vol. 64, November 1975, dispersal of melansomes by prostaglandins is disclosed.

Nordlund, J. J., "Prostaglandin $E_2$ ($PGE_2$) Induces Proliferation of Murine Epidermal Cells", Journal of Investigative Dermatology, Vol, 59, 1985, discloses that pigment cells are seen to increase in numbers after skin is treated with $PGE_2$.

Nordlund, J. J., Collins, C. E., and Rheins, L. A., "Prostaglandin $E_2$ and $D_2$ but not MSH Stimulate the Proliferation of Pigment Cells in the Pinnal Epidermis of the DBA/2 Mouse", Journal of Investigative Dermatology, May 1986, discloses that $E_2$ and $D_2$ have some incremental effect on pigment cell growth and proliferation in mice.

Prostaglandins have also been used for treatment of vascular disorders and skin disorders. U.S. Patent 4,515,810, Chow et al., issued May 7, 1985, incorported herein by reference, discloses topical compositions comprising prostaglandins dissolved in a plasticizer to provide foams for such treatment. U.S. Patent 4,311,707, Birnbaum et al, issued July 19, 1982, also incorporated herein by reference, discloses E and F prostaglandins for topical administration to produce cutaneous vasodilation.

However, because prostaglandins do not readily penetrate the skin, a problem has been encountered in formulating a composition that is both safe and effective. High concentrations of prostaglandins in a topical composition could cause skin irritation and redness as well as other negative effects. The ability to use low levels and yet obtain good performance would be attractive.

The art is replete with disclosures of topical vehicles which are said to improve the penetration of a variety of pharmaceutical actives through the skin. Indeed, it is well recognized that the development of such vehicles would provide a very useful method for the delivery of pharmaceuticals. It has been taught that a binary penetration system comprising specific polar lipid cell-envelope disordering compounds, such as oleic acid or methyl laurate, together with specific diol compounds, such as propylene glycol, can be used to topically deliver systemic levels of selected pharmaceutical agents, such as steroids and non-steroidal anti-inflammatory agents. However, these vehicles have not been found to be equally effective for enhancing the delivery of all pharmaceutical agents.

In order to be useful as a topical vehicle for an artificial tanning agent, the vehicle must satisfy four stringent requirements:

(1) the vehicle must provide enhanced penetration of the agent through the skin;

(2) the vehicle must have cosmetically-acceptable aesthetics;

(3) the vehicle must not be irritating to skin; and

(4) the vehicle must be preferentially substantive to skin rather than to, for example, clothing. While some vehicles may satisfy several of these criteria, the best vehicles will be optimum for all of them.

European Patent Application 43,738, published January 13, 1982, describes compositions for topical application. The compositions described are suitable for effective delivery of lipophilic, pharmacologically-active compounds using primary alcohols or various carboxylate compounds in combination with selected diols.

European Patent Application 95,813, published December 7, 1983, discloses a binary penetration system utilizing a diol and a cell-envelope disordering compound to aid in the penetration of 9-hydroxyethoxymethyl (and related) derivatives of 6- 2, 6-substituted purines. These purine compounds are reported to be effective in the treatment of viral infections, especially herpes, and can be administered parenterally, orally or topically. 9-(2-hydroxyyethoxymethyl) guanine is disclosed as being a particularly preferred active.

U.S. Patent 4,552,872, Cooper, et al., issued November 12, 1985, describes topical pharmaceutical compositions containing corticosteroids and a skin penetration enhancing vehicle comprising selected diols and cell-envelope disordering compounds. These compositions are taught to provide systemically active levels of the corticosteroids via topical administration. A similar disclosure is provided in European Patent Application 129,283, published December 27, 1984.

U.S. Patent 4,557,934, Cooper, issued December 10, 1985, describes pharmaceutical compositions for topical application containing any of a wide range of pharmaceutical actives together with a diol compound and 1-dodecyl-azacycloheptan-2-one. See also European Patent 129,284, published December 27, 1984.

U.S. Patent 4,537,776, Cooper, issued August 27, 1985, describes pharmaceutical compositions for topical application containing any of a wide range of pharmaceutical actives together with N-(2-hydroxyethyl) pyrrolidone. A similar disclosure is provided in European Patent Application 129,285, published December 27, 1984.

European Patent Application 171,742, published February 18, 1986, discloses topical compositions which provide systemically active doses of opioids to the user. The penetration enhancing topical vehicle comprises at least one of a saturated fatty acid or fatty alcohol of 8-15 carbon atoms or of an unsaturated fatty alcohol or fatty acid of 8-18 carbon atoms, and a pharmaceutical carrier such as propylene glycol.

1,2-propanediol ("propylene glycol") and the $C_{10}$-$C_{14}$ alcohols have been used, separately, in cosmetic and pharmaceutical formulations. In particular, propylene glycol has been described in several articles in the literature as enhancing the penetration of certain pharmacologically active agents, such as the corticosteroids. See Rosuold, J., et al., "Effect of Formulation on In Vitro Release and In Vivo Absorption of Corticosteroids from Ointments", Medd. Novsk Favm Selsk, 44, 21-45 (1982); see also, Anjo, D.M., et al., "Methods for Predicting Percutaneous Penetration in Man", Percutaneous Absorption of Steroids, pp. 31-51, Academic Press, New York, New York (1980).

U.S. Patent 3,535,422, Cox, et al., October 20, 1970, relates to stable benzoyl peroxide compositions

containing organic emollients selected from $C_4$-$C_{20}$ aliphatic alcohols $C_2$-$C_3$ glycols, $C_{12}$-$C_{20}$ fatty acids and their esters, and mixtures thereof.

U.S. Patent 4,070,462, Ecker, issued January 24, 1978, describes topical steroid compositions containing 6% propylene glycol and 1% propylene glycol monostearate.

Canadian Patent 1,072,009, Sipos, issued February 19, 1980, describes topical antimicrobial compositions containing $C_5$-$C_{10}$ straight chain alcohols or $C_{17}$ branched chain alcohols in which the longest chain is $C_5$-$C_{10}$.

CA 92: 153, 181j describes an indomethacin ointment containing 10% propylene glycol and 1.1% diisopropanolamine.

B. Idson, Cosmetics & Toiletries, 95 59 (1980), states that the factors affecting drug penetration and, consequently in most cases, effectiveness, are complex. He observes that the vehicle that provides ideal penetration conditions for one drug may provide unsatisfactory for another. The author concludes that prediction is not simple and product suitably must be assessed by human trials.

Applicants have surprisingly found that when used in the topical vehicles described herein for enhanced penetration low levels of prostaglandins $E_1$, $E_2$ and $D_2$ will simulate the natural tanning process causing the skin to produce melanin in increasing amounts, and thus produce a darker or tanned appearance without redness, irritation and are thus both safe and effective compositions.

The artificial tanning compositions herein will cause tanning in the absence of ultraviolet exposure. When used in combination with exposure to a source of ultraviolet radiation, tanning occurs more quickly and darker tanning occurs within the periods of exposure than if the artificial tanning composition had not been used.

It is therefore an object of the present invention to provide a method for the treatment of skin by safe and effective amounts of certain prostaglandins in a vehicle for enhanced penetration in order to produce a tanned appearance.

It is a further object of the invention to provide compositions for enhanced penetration of skin by safe and effective amounts of certain prostaglandins in order to provide a tanned appearance.

It is a further object of the present invention to provide topical compositions which are aesthetically pleasing.

This and further objects will become evident from the description of the invention.

All percentages and ratios herein are by weight unless specifically noted.

## Summary of the Invention

A method of the treatment for the tanning of skin wherein an artificial tanning agent selected from the group consisting of prostaglandin $E_1$, prostaglandin $E_2$, and prostaglandin $D_2$ ($PGE_1$, $PGE_2$, and $PGD_2$) or mixtures thereof, is applied to the skin. Topical compositions for such tanning comprise an effective amount of such an artificial tanning agent or mixture thereof, and the following penetration enhancing vehicle:

a) 10% to about 40% of a diol or triol compound,

b) from about .05% to about 3% of a polar lipid compound, and

c) from about 1% to about 80% of a solvent.

## Detailed Description

### Artificial Tanning Agents

Prostaglandins are a large group of closely related carboxylic acids containing a cyclopentane ring with two adjacent carbon chains. One carbon chain has the carboxyl group in the terminal position. Prostaglandins are divided into five types (A-F) based on the functional groups in the cyclopentane ring. Numerical subscripts refer to the number of unsaturations in the side chains.

The prostaglandins useful in compositions of the invention are $PGE_1$, $PGE_2$, and $PGD_2$ they have the following structures:

PGE$_1$

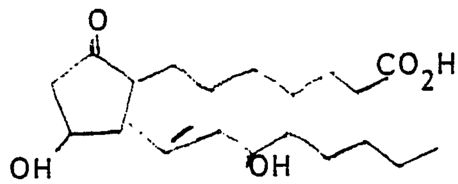

PGE$_2$

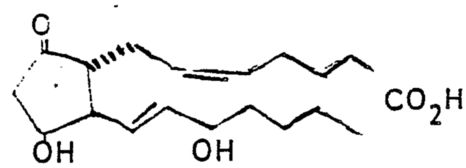

PGD$_2$

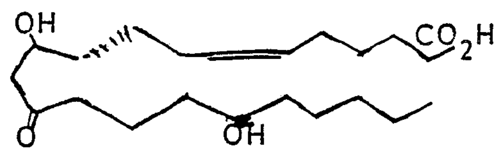

$E_1$ and $E_2$ are two of the six naturally-occurring primary prostaglandins in that they are not derived from another prostaglandin. E types used herein may be naturally derived or synthetic analogs. Naturally occurring prostaglandins may be regarded as derivatives of prostanoic acid.

$PGD_2$ is derived from arachidonic acid. Arachidonic acid is an unsaturated 20 carbon chain fatty acid containing four double bonds. It is normally found in the plasma membrane of all mammalian cells, including skin cells.

Prostaglandins of the E-type and esters thereof have been widely used as pharmacological actives such as antilipodemics, hypotensives, broncodilators, fertility control agents, and gastric secretion inhibitors. See e.g., U.S. Patent 3,069,322, Bergstrom, issued December 18, 1962 and U.S. Patent 3,598,858, Bergstrom, issued August 10, 1971.

Applicants have discovered that prostaglandins $E_1$, $E_2$ and $D_2$, when applied in topical compositions of the invention at a concentration of about 0.1 microliter/sq. centimeter of skin to about 50 microliters/sq. centimeter of skin, preferably from about 1 microliter/sq. centimeter to about 10 microliters/sq. centimeter will result in the skin acquiring a tanned appearance in the absence of exposure to ultraviolet radiation. A darker, faster tanned appearance will result if the prostaglandins are applied to skin which is concurrently or subsequently exposed to an ultraviolet radiation source.

Without wishing to be bound by theory, it is believed that the prostaglandins stimulate melanogenesis on the dermal layer of the skin, causing an increased production of melanin. The increased melanin gives the skin a darkened or tanned appearance without the erythema which accompanies an ultraviolet source induced tanned appearance. It is further believed that the elimination of concurrent skin damage through use of artificial tanning agents may reduce long term skin damage including, but not limited to, wrinkling, skin toughening, elastosis and skin cancer, all of which are induced or exacerbated by ultraviolet exposure.

## Vehicle

The penetration vehicle for the prostaglandins to be topically introduced to the skin for treatment comprises a solvent, a polar lipid compound and a diol or triol compound.

The solvent for the prostaglandins comprises from about 1% to about 80% of the composition. Ethanol or 2-propanol (isopropanol or isopropyl alcohol) are preferred solvents for the prostaglandin and preferably

comprise from about 10% to about 75% of the composition. Other solvents such as ethyl acetate, acetone and other primary alcohols, may also be present. Where such other solvents are present, they comprise from about 40% to about 65% of the composition.

A short chain diol or triol compound serves as a penetration enhancer and comprises from about 5% to about 40% of the composition. $C_3$-$C_4$ diols useful in the compositions to enhance penetration include 1,3-propanediol, 1,2-butanediol, 1,3-butanediol, 1,4-butanediol or mixtures thereof. The $C_3$-$C_6$ triol compounds useful in compositions of the invention include glycerol, 1, 2, 6-hexane-triol, 1, 2, 5-pentanetriol 1, 2, 4-butanetriol, 1, 2, 5-hexanetriol or mixtures thereof. Mixtures of diol and triol compounds may also be used. 1,2-Propandiol (or propylene glycol) is preferred.

Compositions of the invention additionally comprise from about 0.05% to about 3% of a polar lipid compound. Preferred polar lipids are selected from the group consisting of oleyl alcohol, oleic acid, methyl laurate, myristyl alcohol, and mixtures thereof. The preferred polar lipid compounds enhance skin penetration of the active, do not irritate the skin, and are substantive to the skin. This is particularly surprising since the most preferred compound, oleyl alcohol, has been taught in the art to be irritating to the skin when topically applied. Preferably the compositions comprise from about 0.1% to about 1% of such polar lipid compound.

## Optional Ingredients

The compositions of the present invention may additionally contain adjunct components conventionally found in topical pharmaceutical and/or cosmetic compositions such as astringents, antimicrobials, anti-inflammatory agents, excipients, emulsifiers, emollients, dyes, perfumes, preservatives, anti-oxidants, opacifiers, thickeners and stabilizers. Such materials should not interfere with the penetration of the compound. Preferred optional ingredients are those which give improved aesthetics to the composition.

In preferred embodiments of the invention, the compositions are emulsions. They may be water-in-oil emulsions or oil-in-water emulsions, and the viscosities may range from that of a thin lotion to that of a very thick cream. In these embodiments, emulsifiers are highly preferred optional ingredients.

Emulsifiers suitable for use can be any of a wide variety disclosed in the prior patents and other references. Two patents, incorporated by reference herein, which disclose suitable emulsifiers are U.S. Patent 3,755,560, August 28, 1973 to Dickert et al. and U.S. Patent 4,421,769, December 20, 1983 to Dixon et al. Preferred emulsifiers are anionic or nonionic although other types may also be used. Another reference disclosing an extensive number of emulsifiers is McCutcheon's Detergents & Emulsifiers, North American Edition, 1983, incorporated herein by reference.

Suitable emulsifier types include ethoxylated fatty acids, ethoxylated esters, ethoxylated ethers, ethoxylated alcohols, phosphated esters, polyoxyethylene fatty ether phosphates, fatty acid amides, acyl lactylates, soaps and mixtures thereof. Fatty alcohols such as cetyl and stearyl alcohol and cetearyl alcohol are also regarded as emulsifiers for purposes of the present invention.

Examples of such emulsifiers include polyoxyethylene (8) stearate, myristyl ethoxy (3) myristate, polyoxyethylene (100) monostearate, lauric diethanolamide, stearic monoethanolamide, hydrogenated vegetable glycerides, sodium stearoyl-2-lactylate and calcium stearoyl-2-lactylate. Soaps are also acceptable emulsifiers. The soaps may be formed in situ in processing the composition and are preferably alkali metal or triethanolamine salts of long chain fatty acids. Such soaps include sodium stearate, triethanolamine stearate and the similar salts of lanolin fatty acids.

Optional oil soluble materials may also be present, including nonvolatile silicone fluids such as polydimethyl siloxanes with viscosities ranging from about 10 to about 100,000 centistokes at 25°C. These siloxanes are available from Dow Corning Corporation as the Dow Corning 200 series.

Other oil soluble materials include esters such as cetearyl palmitate, lauryl myristate and isopropyl palmitate; oils such as castor oil, jojoba oil, cottonseed oil, peanut oil and sesame oil; waxes such as petrolatum, cersin wax, carnauba wax, beeswax, and castor wax; lanolin, its derivatives and components such as acetylated lanolin, lanolin alcohols and lanolin fatty acids. Sterols such as cholesterol and phytosterol are also useful herein.

These optional oil phase materials may individually comprise up to about 20% of the total composition, preferably up to about 10%.

Additional water soluble materials may also be present in the compositions of this invention. Included are humectants such as sorbitol, alkoxylated glucose and tyrosine; thickening agents such as carboxyvinyl polymers (Carbopols® - offered by B. F. Goodrich Company, such polymers are described in detail in U.S. Patent 2,798,053, July 2, 1957 to Brown, incorporated herein by reference); ethyl cellulose, polyvinyl

alcohol, carboxymethyl cellulose, vegetable gums and clays such as Veegum® (magnesium aluminum silicate, R. T. Vanderbilt, Inc.); proteins and polypeptides; preservatives such as the methyl, ethyl, propyl and butyl esters of hydroxybenzoic acid (Parabens - Mallinckrodt Chemical Corporation), EDTA, methylisothiazolinone and imidazolidinyl ureas (Germall 115 - Sutton Laboratories); and an alkaline agent such as sodium hydroxide or potassium hydroxide to neutralize, if desired, part of the fatty acids or thickener which may be present.

The optional water phase materials may individually comprise up to about 20% of the total composition, preferably up to about 10%.

The pH of the compositions herein is preferably in the range of from about 4.5 to about 9.

## METHOD OF USE

The compositions of the invention are applied to the skin over that portion of the body where the consumer desires to have a tanned appearance.

The composition should be applied about once or twice daily in an amount that is safe and effective. To be effective, the composition should be applied such that from about 0.1 to about 50 microliters of an active is distributed over a square centimeter of skin. The average body represents about 1.5 square meters of skin.

The amounts of prostaglandin per unit volume of composition required to deposit the amount indicated varies from about 10 µg/ml to about 50 µg/ml and is dependent on several factors including viscosity, aesthetics, etc. The tanned appearance will not occur immediately as with dyed products but will develop over time, as the skin produces melanin.

The composition should be applied evenly over the area which is to be tanned and allowed to penetrate the skin. The treated area of the skin should preferably be kept away from water, i.e. swimming or bathing for about 30 minutes to one hour after application in order to allow it to fully penetrate the skin. Daily application should continue until the desired shade or darkness of tan is achieved. Thereafter, such frequent application is not necessary, and application could be reduced to from about once every two days to about once per week or less in order to maintain the desired shade. This will vary depending on skin type, exposure to ultraviolet rays, etc. Compositions of the invention may also be used to maintain a tanned appearance, whether achieved by artificial means or exposure to a source of ultraviolet radiation. The compositions should be applied to the skin as described above from about once per day to about once per week, again, varying depending on skin type, continued exposure, etc.

## METHODS OF MANUFACTURE

Compositions of the invention are prepared by methods described in Examples I and III.

## EXAMPLES

### Example I

The following is a representative composition:

$PGE_1$ 0.01% mg/ml
Oleyl alcohol 0.2%
Propylene glycol 30.0%
Ethanol 69.79%

The prostaglandin ($PGE_1$) is weighed and placed in a glass volumetric cylinder. Ethanol is then added to solubilize the PGE. The propylene glycol and oleyl alcohol are then added.

Example II

The following is a lotion composition representative of the invention.

Prostaglandin $E_1$ 0.01%
Perfume 0.50%
Carbopol 954 0.50%
Oleyl alcohol 0.50%
Cetyl alcohol 1.00%
Propylene glycol 15.00%
Ethanol 30.00%
qs $H_2O$ to 100%

Example III

The following is a cream composition of the invention.

Prostaglandin $E_2$ 0.01%
Perfume 0.05%
Carbopol 934 0.50%
Oleyl alcohol 0.50%
Cetyl alcohol 2.50%
Myristyl alcohol 2.00%
Propylene glycol 15.00%
Ethanol 30.00%
qs $H_2O$ to 100%

The above composition is prepared by:

A) preparing the oil phase; (cetyl alcohol, myristyl alcohol, oleyl alcohol, perfume)
B) preparing the water phase; (propylene glycol, Carbopol, ethanol, water)
C) adding the oil phase to the water phase; and
D) adding the solubilized prostaglandin.

Step A is carried out by heating the oil phase materials to a temperature of from about 75°C to about 100°C. Step B is carried out by heating the water phase materials to a like temperature. The emulsion is formed by slowly adding the oil phase prepared in step A to the water phase prepared in Step B. The prostaglandins are solubilized in a portion of the ethanol and added to the mixed oil and water phases.

In the above compositions, similar results are obtained if the ethanol is replaced by isopropanol, ethyl acetate, acetone or mixtures thereof. Likewise similar results are obtained if prostaglandin $E_2$ is replaced by $PGE_1$ or $PGD_2$. Further, the propylene glycol may be replaced by 1,3-propanidiol, 1,3-butanediol, glycerol, 1,2,6-hexanetriol or mixtures thereof without affecting the results.

## Claims

1. A topical composition for giving the skin a tanned appearance in the absence of ultraviolet radiation exposure or achieving a faster, darker tanned appearance in the presence of ultraviolet radiation exposure comprising:

a) a safe and effective amount of an artificial tanning agent selected from prostaglandins $E_1$, $E_2$, and $D_2$;
b) from 1% to 80% of a solvent for the prostaglandins;
c) from 5% to 40% of a diol or triol compound; and
d) from 0.05% to 3% of a polar lipid compound.

2. A topical composition according to Claim 1 wherein the polar lipid compound is selected from oleyl alcohol, oleic acid, methyl laurate, myristyl alcohol and mixtures thereof.

3. A topical composition according to Claim 2 wherein the polar lipid compound is oley alcohol.

4. A topical composition according to any of Claims 1 to 3 wherein the solvent is selected from the primary alcohols, acetone, ethyl acetate and mixtures thereof.

5. A topical composition according to Claim 4 wherein the solvent is ethanol.

6. A topical composition according to Claim 5 wherein ethanol comprises from 10% to 80% of the composition.

7. A topical composition according to any of Claims 1 to 6 wherein the diol or triol compound is selected from 1,2-propanediol, 1,2-butanediol, 1,2,6-hexanetriol and glycerol.

8. A topical composition comprising:

a) from 10µg/ml to 500 µg/ml of an artificial tanning agent selected from prostaglandin $E_1$, prostaglandin $E_2$, and prostaglandin $D_2$;

b) from 20% to 60% ethanol;

c) from 10% to 35% propylene glycol; and

d) from 0.1% to 1% oleyl alcohol.

9. A topical composition according to Claim 8 comprising from 40% to 65% of an additional solvent selected from primary alcohols, acetone, ethyl acetate and mixtures thereof.

10. A topical composition according to any of Claims 1 to 9 further comprising from 0.1% to 10% of an emulsifier.

11. A topical composition according to Claim 10 wherein the emulsifier is selected from fatty alcohols, fatty acids, soap and mixtures thereof.

12. A topical composition comprising:

a) from 10µg/ml to 500 µg/ml of an artificial tanning agent selected from the group consisting of prostaglandin $E_1$, prostaglandin $E_2$ and prostaglandin $D_2$,

b) from 20% to 40% ethanol,

c) from 10% to 20% propylene glycol,

d) from 0.1% to 1% oleyl alcohol,

e) from 0.5% to 10% cetyl alcohol and

f) from 0.1% to 5% of a carboxyl vinyl polymer.

European Patent Office

# EUROPEAN SEARCH REPORT

Application number

EP 87 30 6848

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACT, vol. 87, no. 1, 4th July 1977, page 63, column 1, abstract no. 16212c, Columbus, Ohio, US; J.J. SAUK et al.: "The action of prostaglandins, precursor, and prostaglandin endoperoxide on melanocyte-keratinocytes during anagen stage of human pigmented and albino hair", & PIGM. CELL. 1976, 3, 244-253 |  | A 61 K 31/557<br>A 61 K 7/42<br>A 61 K 47/00 |
|  | --- |  |  |
| A | CHEMICAL ABSTRACTS, vol. 104, no. 22, 2nd June 1986, page 184, column 2 - page 185, column 1, abstract no. 201112c, Columbus, Ohio, US; J. NORDCUND et al.: "Prostaglandin E2 and D2 but not MSH stimulate the proliferation of pigment cells in the pinnal epidermis of the DBA/2 mouse", & J. INVEST. DERMATOL. 1986, 86(4), 433-437 (Cat. D) |  | **TECHNICAL FIELDS SEARCHED (Int. Cl.4)**<br>A 61 K 31/00<br>A 61 K 7/00<br>A 61 K 47/00 |
|  | --- |  |  |
| A | CHEMICAL ABSTRACT, vol. 80, no. 17, 29th April 1974, column 1, abstract no. 104221r, Columbus, Ohio, US; J. ABRAMOWITZ et al.: "In vitro effects of prostaglandins upon melanosome dispersion in the skin of black goldfish, Carassius auratus", & PROSTAGLANDINS 1973, 4(6), 805-818 |  |  |
|  | --- -/- |  |  |

The present search report has been drawn up for all claims

| Place of search | Date of completion of the search | Examiner |
|---|---|---|
| BERLIN | 19-10-1987 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

EP 87 30 6848



## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | EP-A-0 043 738 (PROCTER & GAMBLE)<br>* claims 1, 5, 6 *<br>--- | | |
| A,D | EP-A-0 095 813 (PROCTER & GAMBLE)<br>* claims 1, 3, 4, 10 *<br>----- | | |
| | | | TECHNICAL FIELDS SEARCHED (Int. Cl.4) |

The present search report has been drawn up for all claims

| Place of search | Date of completion of the search | Examiner |
|---|---|---|
| BERLIN | 19-10-1987 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82